Europäisches Patentamt
European Patent Office
Office européen des brevets

(11) Numéro de publication: **0 272 971 A2**

(12) **DEMANDE DE BREVET EUROPEEN**

(21) Numéro de dépôt: **87402756.8**

(22) Date de dépôt: **04.12.87**

(51) Int. Cl.4: **C 07 D 251/34**

(30) Priorité: **11.12.86 FR 8617329**

(43) Date de publication de la demande: **29.06.88 Bulletin 88/26**

(84) Etats contractants désignés: **AT BE CH DE ES GB GR IT LI LU NL SE**

(71) Demandeur: **Société Chimique des Charbonnages S.A.**
**Tour Aurore Place des Reflets**
**F-92080 Paris La Défense 2 Cédex 5 (FR)**

(72) Inventeur: **Teissier, Rémy**
**4, rue des Couteliers**
**F-31000 Toulouse (FR)**

**Clamens, Serge**
**19, rue de la Camargue**
**F-31170 Tournefeuille (FR)**

(74) Mandataire: **Rieux, Michel**
**SOCIETE CHIMIQUE DES CHARBONNAGES S.A. Service Propriété Industrielle Tour Aurore Place des Reflets Cédex no. 5**
**F-92080 Paris la Défense 2 (FR)**

(54) **Di-bishydroxy de l'acide cyanurique, ses dérivés trisubstitués et leur procédé de fabrication.**

(57) L'invention a pour objet de nouveaux dérivés (I) et (II) de l'acide cyanurique qui ont pour formule respectivement

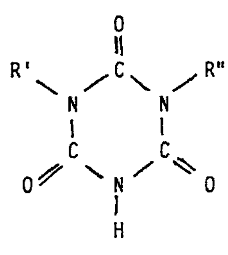

et

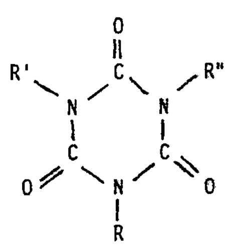

dans lesquelles :
- R′ et R″ sont $-CH_2-CH(OH)-CH_2OH$ et
- R est un groupe alkyle comportant une fonction éthylénique polymérisable telle qu'un groupe acrylique, méthacrylique ou allylique.

Les dérivés (I) et (II) selon l'invention peuvent être utilisés comme agents de réticulation dans les polymères.

EP 0 272 971 A2

**Description**

La présente invention a pour objet de nouveaux dérivés de l'acide cyanurique qui sont le 1,3 di(bishydroxypropyle) isocyanurate, des dérivés trisubstitués de l'acide cyanurique et leur procédé de fabrication.

On connait de nombreux dérivés organiques de l'acide cyanurique. Ce sont des dérivés triaziniques substitués sur l'azote de formule :

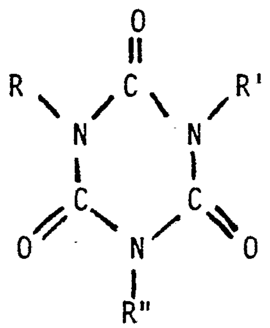

Parmi les dérivés connus de l'acide cyanurique, on peut citer le 1,3,5-tris(3chloro 2hydroxy propyle) isocyanurate dans lequel

R = R′ = R″ = $-CH_2-CH(OH)-CH_2Cl$

Ce dérivé peut être utilisé comme intermédiaire réactionnel dans la préparation du triglycidyle isocyanurate. Il peut être obtenu par réaction de l'acide cyanurique, du formol et de l'épichlorhydrine (brevet polonais N°99060/1975).

On connait également le triallyle isocyanurate dans lequel R, R′ et R″ sont des radicaux $CH_2 = CH - CH_2$ -. On le prépare par action du chlorure d'allyle sur le cyanurate trisodique. Il est utilisé comme réticulant en particulier dans les polyesters.

Des dérivés trisubstitués sur l'azote de l'acide cyanurique, dans lesquels les radicaux R, R′, R″ sont différents, sont également connus. Parmi ceux-ci, on peut citer le diallyle monoacryloyle oxyéthyle isocyanurate dans lequel :

R = R′ = $CH_2 = CH - CH_2$ -

et R″ = $CH_2 = CH - C(O) - O - CH_2 - CH_2$ -

Ces dérivés sont notamment décrits dans le brevet français N° 2 560 874. Ils peuvent être polymérisés ou copolymérisés avec des monomères acryliques ou encore être utilisés comme agent de réticulation dans les polymères tels que les polyéthylènes, les polystyrènes. Ils confèrent à ces derniers des propriétés ignifuges.

La présente invention a pour premier objet un nouveau dérivé (I) de l'acide cyanurique qui est le 1-3 di(bishydroxypropyle)isocyanurate et de formule :

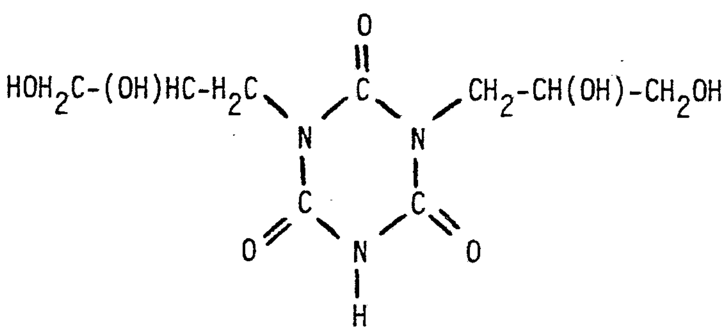

Le spectre infra rouge (figure 1) et le spectre de résonnance magnétique du carbone $C^{13}$(RMN$C^{13}$) (figure 2) confirment la structure du dérivé (I), à savoir le 1-3 di(bishydroxypropyle) 1-3-5 triazine (1H, 3H, 5H) 2-4-6 trione ou 1-3 di(bishydroxypropyle) isocyanurate. Sa formule brute est $C_9H_{15}N_3O_7$. Son poids moléculaire est de 277,35. A température ambiante, il se présente comme un produit blanchâtre très visqueux et hygroscopique. Il est soluble dans l'eau et les alcools tels que le méthanol, l'éthanol et le propanol. Par contre, il est insoluble dans l'acétone, les solvants aromatiques, l'éther. Le 1-3 di(bishydroxypropyle) isocyanurate (I) peut être préparé de diverses manières. Par exemple, il peut être obtenu à partir du 1-3 di(chlorohydroxypropyle) isocyanurate par une réaction d'hydrolyse alcaline. On utilise de préférence un milieu réactionnel de pH au moins égal à 10, de préférence au moins égal à 11, obtenu par exemple à l'aide de la soude ou de la potasse aqueuse. Le rapport molaire de fonctions basiques $OH^{(-)}$ sur le di(chlorohydroxypropyle) isocyanurate est de 3 au moins.

De préférence, la température du milieu réactionnel est comprise entre l'ambiante et 50° C afin d'éviter la réaction d'hydrolyse du cycle cyanurique.

En fin de réaction, on récupère le 1-3 di(bishydroxypropyle) isocyanurate par extraction à l'aide de l'épichlorhydrine, ou de l'éthanol, ou de l'acétonitrile.

La présente invention a pour second objet les dérivés (II) trisubstitués de l'acide cyanurique qui peuvent être obtenus à partir du 1-3 di(bishydroxypropyle) isocyanurate et qui ont pour formule :

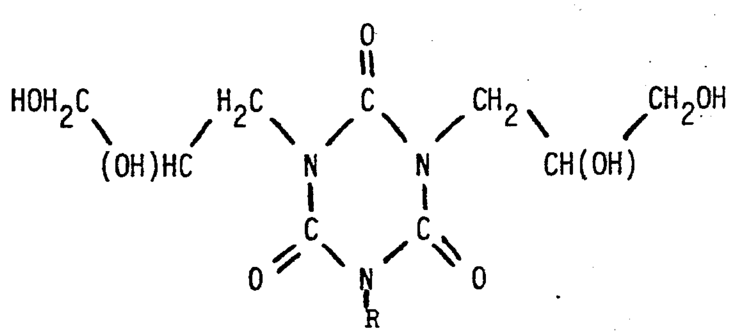

dans laquelle :
- R est un groupe alkyle comportant une fonction éthylénique polymérisable telle qu'un groupe acrylique, méthacrylique ou allylique.

Les dérivés (II) selon l'invention se présentent généralement comme des produits visqueux incolores qui cristallisent à basse température, à environ -30° C.

Les produits (II) selon l'invention ainsi que le 1-3 di(bishydroxypropyle) isocyanurate, peuvent être utilisés comme réticulants dans les polymères tels que les polyoléfines, les polyacryliques, les polystyrènes, les polyallyliques et les polycondensats tels que les polyesters et les époxydes.

Ils confèrent à ces polymères des propriétés telles qu'une bonne résistance à la chaleur et aux U.V.

De plus, ils rendent ces polymères hydrosolubles.

Les produits (II) peuvent également être polymérisés ou copolymérisés avec des monomères tels que les monomères (méth)acryliques, les monomères allyliques, les momomères éthyléniques tels que l'éthylène, le propylène, le chlorure de vinyle, le styrène.

Pour la préparation des dérivés trisubstitués (II) selon l'invention, on peut songer à utiliser différentes méthodes. On peut préparer les dérivés trisubstitués (II) en faisant réagir la 1-3 di(bishydroxypropyle) isocyanurate avec un halogènure RX, X étant un halogène et R étant un groupe alkyle comportant une fonction éthylénique polymérisable telle qu'un groupe acrylique, méthacrylique ou allylique. La réaction est effectuée en présence d'au moins un catalyseur de type de transfert de phase, par exemple les ammoniums quaternaires, à une température comprise entre 50 et 140° C.

La réaction est généralement effectuée à une température comprise entre 50 et 100° C. De préférence, elle est menée à la température de reflux de l'halogénure ou à celle de l'eau dans le cas où la température d'ébullition de l'halogénure est supérieure à 100° C. La réaction peut également être effectuée à une température comprise entre 100 et 140° C, mais dans un autoclave. Au-delà de 140° C, les dérivés (II) selon l'invention se dégradent, et à une température inférieure à 50° C la cinétique de réaction est très faible.

En fin de réaction, on isole et on purifie le dérivé trisubstitué (II) obtenu par extraction à l'aide d'un solvant tel que l'acétone.

De préférence, on utilise un rapport molaire de 1-3 di(bishydroxypropyle) isocyanurate sur l'halogénure égal à 1 ou légèrement inférieur de manière à bénéficier d'un léger excès d'halogénure.

De préférence encore, dans le but d'avoir un milieu réactionnel de faible viscosité, la réaction est effectuée avec des concentrations aqueuses en 1-3 di)(bishydroxypropyle) isocyanurate et en halogénure n'excédant pas 0,5 mole/litre.

Parmi les halogènures d'alkyle utilisables dans le procédé selon l'invention, on peut citer, l'acrylate de chloroéthyle, l'acrylate de chlorométhyle, le méthacrylate de chloroéthyle, le méthacrylate de chlorométhyle.

Parmi les catalyseurs utilisables dans le procédé selon l'invention, on peut citer, par exemple, le chlorure de tétraéthyle ammonium, le bromure de tétraéthyle ammonium, le chlorure de tétraéthyle benzyle ammonium, le bromure de tétraéthyle benzyle ammonium, les amines tertiaires telles que la triméthyle amine, la triéthyle amine ou les sels de phosphonium quaternaire tels que les sels de phosphonium quaternaire, par exemple le bromure de méthyle phenyl phosphonium. Le catalyseur est utilisé de préférence dans un rapport molaire par rapport à la 1-3 di(chlorohydroxypropyle) isocyanurate compris entre 10 $^{-2}$ et 10 $^{-1}$.

**Exemple 1** : Préparation 1-3 di(bishydroxypropyle) isocyanurate

Après avoir fait réagir l'acide trichlorocyanurique avec le diallyle isocyanurate, on filtre l'acide cyanurique libéré. Sous agitation, on ajoute à la solution aqueuse claire contenant 0,5 mole (157g) de 1-3 di(chlorohydroxypropyle) isocyanurate, 62,5g de NaOH en pastilles à 96% (3/2 moles). La température s'élève à 35° C maximum du fait principalement de la dissolution de la soude. On laisse sous agitation durant au moins 16 heures. Au bout de 24 heures, 95% de la stoechiométrie (1 mole) de soude a été consommée, tandis que 98% de la stoechiométrie en NaCl est apparue (dosage acidimétrique - dosage Cl(-)par argentimétrie). La température du mélange réactionnel est alors retombée à l'ambiante (temps t = référence utilisée dans les exemples 4 et 5 suivants). On neutralise à Ph 4, puis on concentre sous vide jusqu'à 0,5 litre environ. On y ajoute 0,5 à 1 litre d'acétone. Un dépôt blanchâtre visqueux apparaît que l'on sépare par centrifugation. Ce dépôt (ci-après dénommé produit A) est repris par l'épichlorhydrine. Le NaCl précipité par l'acétone en même temps que le 1-3 di(bishydroxypropyle) isocyanurate est filtré. On récupère 58,5g d'un produit visqueux translucide très hygroscopique. On a donc récupéré 55g de 1-3 di(bishydroxypropyle) isocyanurate soit 40% de rendement par rapport diallyle isocyanurate. Un dosage argentimétrique montre qu'il y a 6% de NaCl environ dans le produit, ce que confirme l'analyse élémentaire suivante :

| | mesuré | ramené à 100% | théorique |
|---|---|---|---|
| C : | 36,54% | 38,64% | 38,91% |
| H : | 5,5% | 5,82% | 5,42% |
| N : | 14,00% | 14,81% | 15,16% |
| O : | 38,52% | 40,73% | 40,43% |
| Somme : | 94,56% | 100 % | 100 % |

Le spectre RNM (figure 1) et le spectre infra-rouge (figure 2) prouvent la structure du produit. Sur le spectre infra-rouge (figure 2 :

Massif entre 3500 - 3200 cm $^{-1}$ correspond à OH-NH
Pics à 2940 cm $^{-1}$ et 2860 cm $^{-1}$ correspondent à $CH_2$.
Pics à 1770 cm $^{1}$ correspondent à CO
Pics à 1460 cm$^{1}$ correspondent au cycle cyanurique
Bandes vers 1100 cm $^{-1}$ correspondent à C-OH et aux glycoles
Bande à 760 cm $^{-1}$ correspond au cycle cyanurique

**Exemple 2** : Préparation du 1-3 di(bishydroxypropyle) isocyanurate

Le produit (A) obtenu dans l'exemple I est repris par l'éthanol absolu que l'on évapore. On récupère 165g d'un produit visqueux qui contient 34% environ de NaCl. Le spectre infra-rouge est identique à celui obtenu dans l'exemple 1 (figure 2). Dans ce mélange il y a 110g de di(bishydroxypropyle)isocyanurate soit 80% de rendement par rapport au diallyle isocyanurate.

Une seconde extraction à l'éthanol conduit à 96g d'un produit qui contient 10,6% de NaCl, c'est-à-dire un rendement de 61% par rapport au diallyle isocyanurate.

**Exemple 3** : Préparation du 1-3 di(bishydroxypropyle isocyanurate

L'extraction du produit (A) est faite à l'aide de méthanol. Après 3 extractions, on obtient 103g d'un produit visqueux hygroscopique qui contient 15% environ de NaCl et qui a un spectre infra-rouge indentique aux précédents (se reporter à la figure 2).

**Exemple 4** : Préparation du 1-3 di(bishydroxypropyle) 5 monoallyle isocyanurate

Pour la préparation de ce composé, on utilise le mélange réactionnel préparé dans l'exemple 1, non purifié et non neutralisé et tel qu'obtenu au temps t noté dans cet exemple. A ce mélange réactionnel on ajoute :

- 39g de chlorure d'allyle (0,51 mole)
- 5g d'un produit de dénomination commerciale Triton B (solution aqueuse à 40% de Benzyltriméthyl ammonium hydroxyde).

On porte le mélange au reflux qui débute vers 48° C. A mesure que le chlorure d'allyle réagit, la température de reflux s'élève. Au bout de 4 heures elle se stabilise vers 95,6° C. On refroidit. On dose les ions chlorures par argentimétrie. Environ 0,45 mole de Cl(-) sont apparus durant cette réaction soit un taux de réaction de 90%. On concentre sous vide. On obtient un pâté sur lequel on effectue 3 extractions à l'acétone. On évapore l'acétone. On obtient 602 g de produit visqueux de 1-3 di(bishydroxypropyle) 5 allyle isocyanurate (rendement par rapport au diallyle : 38%).

L'analyse complémentaire confirme le produit :

C : 44,82% (th 45,43%
H : 6,14% (th 5,99%)
N : 12,95% (th 13,25%)

O : 35,87% (th 35,33%)

Ce produit est soluble dans l'eau, les alcools, l'acétone. Il est insoluble dans l'éther et les aromatiques.

**Revendications**

**1** - Dérivé de l'acide cyanurique de formule (I)

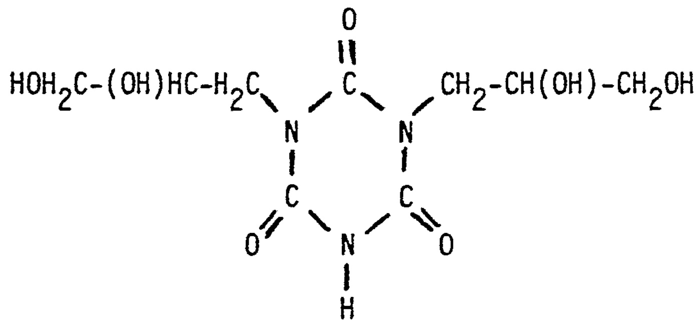

**2**- Procédé de préparation du dérivé (I) défini dans la revendication 1, caractérisé en ce que l'on fait réagir du 1-3 di(chlorohydroxypropyle) isocyanurate par une réaction d'hydrolyse alcaline, le pH du milieu réactionnel étant d'au moins égal à 10, et en ce que l'on récupère ensuite le dérivé (I) ainsi obtenu.

**3** - Procédé selon la revendication 2, caractérisé en ce que le pH du milieu réactionnel est au moins égal à 11.

**4** - Procédé selon la revendication 2 ou 3, caractérisé en ce que la température de réaction est comprise entre l'ambiante et 50° C.

**5** - Dérivés trisubstitués de l'acide cyanurique caractérisés en ce qu'ils ont pour formule (II)

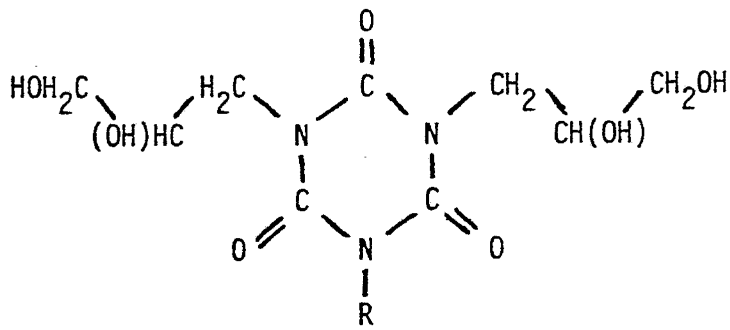

dans laquelle R est un groupe alkyle comportant une fonction éthylénique polymérisable telle qu'un groupe acrylique, méthacrylique ou allylique.

**6** - Procédé de préparation des dérivés de formule (II) définis dans la revendication 5, caractérisés en ce qu'on fait réagir le dérivé (I) défini dans la revendication 1 avec un halogénure de formule RX, X étant un halogène et R étant un groupe alkyle comportant une fonction éthylénique polymérisable, en présence d'au moins un catalyseur de type de transfert de phase à une température comprise entre 50 et 140° C, et en ce que l'on sépare le dérivé (II) ainsi obtenu.

**7** - Application du dérivé (I) et des dérivés (II) respectivement définis dans les revendications 1 et 5 comme agent réticulant dans les polymères.

FIGURE 1

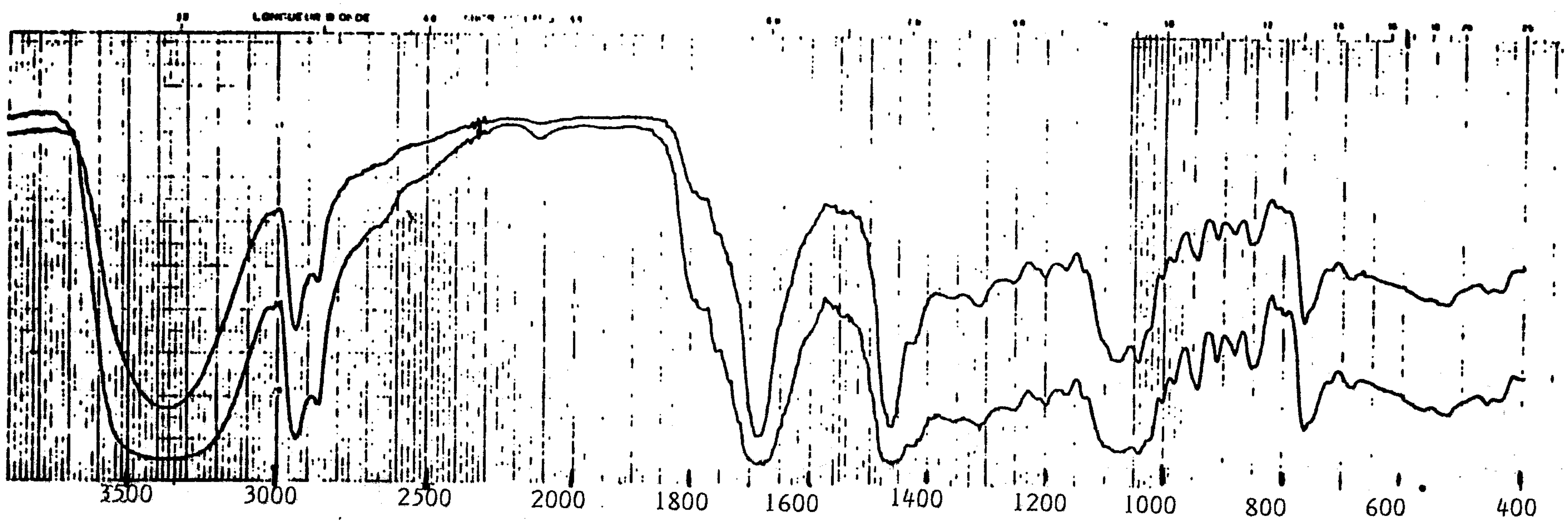
LONGUEUR D'ONDE
3500
3000
2500
2000
1800
1600
1400
1200
1000
800
600
400

29.01.88

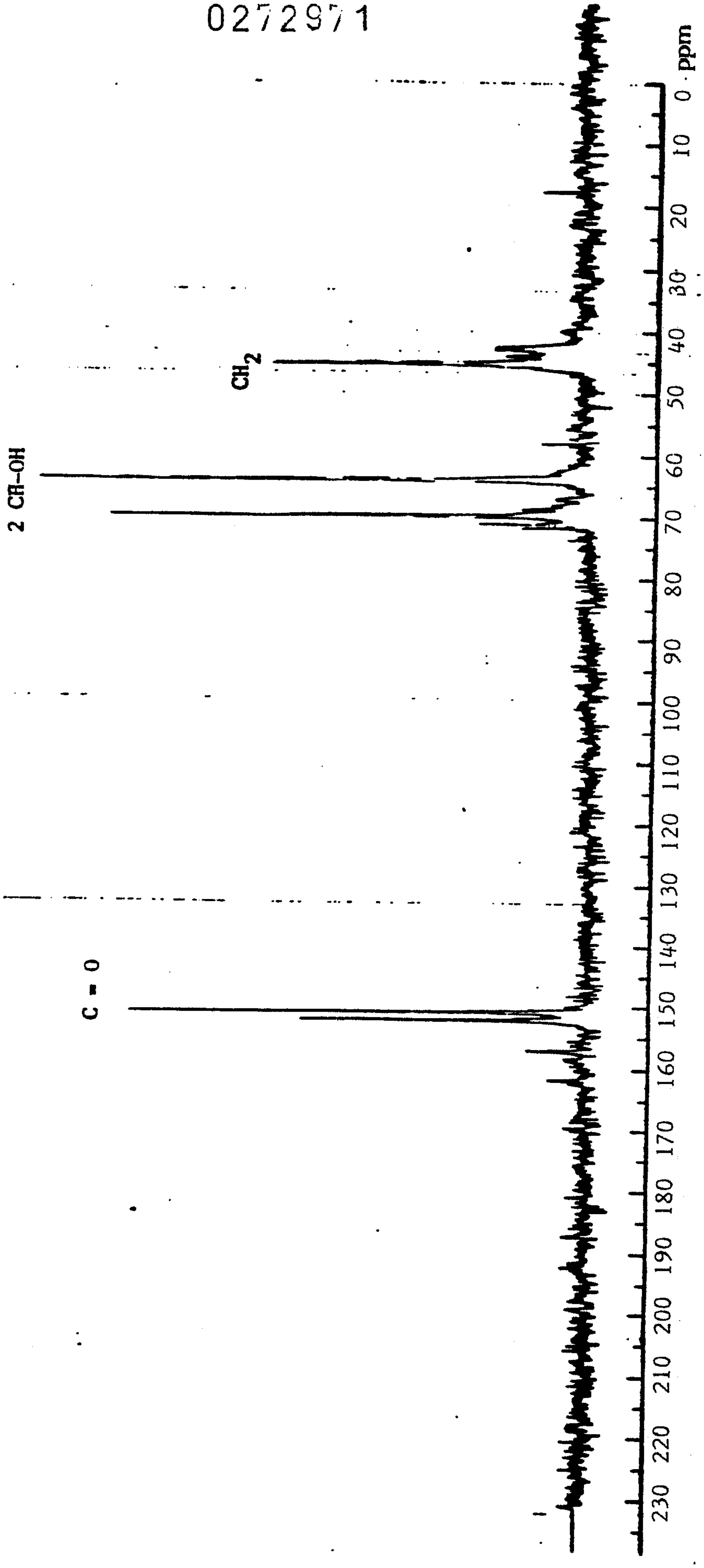


FIGURE 2